# EUROPÄISCHE PATENTSCHRIFT

(11) **EP 2 113 079 B1**
(45) Veröffentlichungstag und Bekanntmachung des Hinweises auf die Patenterteilung: **01.08.2018**
(21) Anmeldenummer: 08707030.6
(22) Anmeldetag: 14.01.2008
(51) Int. Cl.: G01N 27/411, C21C 1/04, C21C 1/08, C21C 7/00, F27D 21/00, C21C 5/46, G01N 33/20

(54) **Sensor zum Messen des Sauerstoffgehaltes in Gusseisenschmelzen**
Sensor for determining the oxygen content of cast iron
Capteur pour la détermination d'oxygène de la fonte

(30) Priorität: 22.01.2007 DE 102007004147
(43) Veröffentlichungstag der Anmeldung: 04.11.2009
(60) Teilanmeldung: 10005996.3
(73) Patentinhaber: Heraeus Electro-Nite International N.V., 3530 Houthalen (BE)
(72) Erfinder: HABETS, Danny, B-3600 Genk (BE)
(74) Vertreter: Heraeus IP
(86) Internationale Anmeldenummer: PCT/EP2008/000226
(87) Internationale Veröffentlichungsnummer: WO 2008/089894

(56) Entgegenhaltungen:
- EP-A- 0 363 616
- EP-A- 1 143 023
- DE-A1- 4 135 510
- DE-B3- 10 310 387
- JP-A- 1 173 863
- JP-A- 57 149 956
- US-A- 4 014 686
- US-A- 4 657 641
- US-B1- 6 544 359
- GOMYO K ET AL: "THREE-PHASE ZIRCONIA SENSOR FOR RAPID DETERMINATION OF SILICON LEVELS IN HOT METAL" TRANSACTIONS OF THE IRON & STEEL SOCIETY, WARRENDALE, US, 1. März 1993 (1993-03-01), Seiten 87-95, XP002913082 ISSN: 1051-0508

## Beschreibung

Die Erfindung betrifft einen Sensor zum Messen des Sauerstoffgehaltes in Gusseisenschmelzen mit einer elektrochemischen Messzelle, die ein Festelektrolytröhrchen aufweist.

Allgemein wird der freie Magnesiumgehalt in einer Gusseisenachmelze als bestimmender Faktor angesehen für die Bildung von sphäroidischem oder vermikularem Graphit im magnesiumbehandeltem Gusseisen. Die gegenwärtige Praxis zur Regelung der Produktion von duktilem Gusseisen besteht in der Bestimmung des Gesamt-Magnesiumgehaltes, das heißt, des freien und des gebundenen Magnesiums, mit Hilfe von spektrographisch untersuchten Proben. Diese Methode ergibt allerdings ein unvollständiges Bild, da der Gehalt an freiem Magnesium nicht bekannt ist und die Messung keine Information über die Sauerstoffaktivität ergibt. Die Sauerstoffaktivität, die im Gleichgewicht mit dem freien Magnesium ist, ist jedoch ein bestimmender Faktor in der Formierung der Graphitform. Das sogenannte duktile Gusseisen ist normaler Grauguss, der mit einem eine Kugel bildenden Zusatz behandelt ist, so dass der Hauptteil des graphitischen Kohlenstoffs im Gusselsen sogenannter Nodulargraphit (Knötchengraphit) oder kugelförmiger Graphit ist. Nodulargraphit in Gusseisen muss hinsichtlich Form, Größe und Teilchenzahl analysiert werden, da diese Kenngrößen die mechanischen Eigenschaften des Gusseisens beeinflussen. Eine visuelle Analyse ist komplex oder subjektiv, selbst bei teilautomatisierten Analysen. Messungen hierzu sind beispielsweise aus US 5,675,097 bekannt. In der DE 199 28 456A1 sind Messungen zur Bestimmung der räumlichen Struktur von Graphit in Gusseisen beschrieben, die auf eine Sauerstoffbestimmung basieren und die Nachteile visueller Methoden nicht aufweisen. Dadurch kann schneller reagiert werden und die gezielte Beeinflussung der Produktion erhöht die Ausbeute bzw. senkt den Ausschuss beim Gießen. Die Qualität des Gusseisens wird gut regelbar.

Der Erfolg der Magnesiumbehandlung in Gusseisen kann beispielsweise durch metallographische oder spektrographische Analysen von weißerstarrten Proben oder auch durch thermische Analysen erfolgen.

Allgemein wird reines Magnesium oder eine Magnesiumlegierung genutzt, um die Kugelform des Gusseisens zu fördern. Ein Teil des zugefügten Magnesiums entzieht dem Eisen Sauerstoff und Schwefel, der verbleibende Teil ist der sogenannte freie Magnesiumanteil, der die Sauerstoffaktivität regelt. Der freie Magnesiumgehalt in der Schmelze ist der bestimmende Faktor für die Nodularität des Gusseisens. Der freie Magnesiumanteil nimmt in der Schmelze im Laufe der Zeit ab, während die Sauerstoffaktivität steigt. Dies beeinflusst die Struktur und mechanischen Eigenschaften des Gusseisens.

Sensoren zur Bestimmung der Sauerstoffaktivität einer Metallschmelze sind beispielsweise aus DE 103 10 387 B3 bekannt. Hier ist ein Festelektrolytröhrchen offenbart, das an seiner äußeren Oberfläche eine Beschichtung aus einer Mischung aus Kalziumzirkonat und einem Fluorid aufweist, so dass beispielsweise in Eisenschmelzen die Messung der Konzentration von Schwefel, Silizium oder Kohlenstoff erfolgen kann. Aus JP 01 173863 A ist ein Sensor bekannt, bei dem zum Messen in Gusseisenschmelzen auf dem Festelektrolyten eine Schicht aus verschiedenen Oxiden und Fluoriden angeordnet ist. Auch aus DE 103 10 387 B3 ist ein Sauerstoffsensor bekannt mit einer Beschichtung in stöchiometrischer Zusammensetzung. JP 57 149956 A beschreibt einen Sensor, der zur Verbesserung der Benetzbarkeit an seiner Oberfläche eine Pulvermischung aus Metalloxiden, organischem Binder, Metallflooriden und Oxidglas aufweist. US 4 657 641 A offenbart einen Silizium-Sensor mit einer Zusatzelektrode aus im Wesentlichen einer Zweiphasenmischung aus ZrO₂ und ZrSiO₄.

Aufgabe der vorliegenden Erfindung ist es, einen Sensor zur Regelung eines Verfahrens anzugeben, mit dem die bisherige Technik verbessert wird, wobei die mechanischen Eigenschaften des Gusseisen bereits in der flüssigen Phase gezielt beeinflusst werden sollen.

Die Aufgabe wird gelöst durch die Merkmale des unabhängigen Anspruchs. Vorteilhafte Ausgestaltungen sind in den Unteransprüchen angegeben. Ein Verfahren zum Beeinflussen der Eigenschaften von Gußeisen besteht darin, dass der Sauerstoffgehalt der Gusseisenschmelze gemessen wird und dass der Gusseisenschmelze so lange Magnesium zugeführt wird, bis der Sauerstoffgehalt der Gusseisenschmelze bei einer Temperatur von etwa 1.420C° als Referenztemperatur etwa 0,005 bis 0,2 ppm beträgt. Da die Sauerstoffmessung genauer ist als die bisher mögliche Magnesiummessung (Magnesium liegt in der Schmelze als freies Magnesium und als gebundenes Magnesium vor, so dass eine genaue Erfassung nicht möglich ist), ist die Bestimmung der mechanischen Eigenschaften des Gusseisens genauer. Der Fachmann kann eine Korrelation zwischen der Existenz weniger großer Graphitpartikel bei niedrigem Sauerstoffgehalt einerseits und vieler kleiner Graphitpartikel bei höherem Sauerstoffgehalt andererseits feststellen und nutzen.

Damit ist eine Korrelation zu den mechanischen Eigenschaften, wie bereits in US 5,675,097 beschrieben, möglich, wie zum Beispiel hinsichtlich der Bruchfestigkeit, der Dehnung und des Widerstandes gegen Verformungen. Für Gusseisen hat es sich überraschend erwiesen, dass es eine maximale Dehnung dann aufweist, wenn die Magnesiumzugabe solange erfolgt, bis der Sauerstoffgehalt kleiner ist als 0,1 ppm, vorzugsweise zwischen 0,08 und 0,1 ppm. Dei niedrigerem oder höherem Sauerstoffgehalt nimmt die Dehnung des Gusseisens wieder ab. Vorteilhaft ist es, dass etwa 200 bis 750 ppm Magnesium zu der Gusseisenschmelze zugegeben werden, um den gewünschten Sauerstoffgehalt zu erreichen.

Der erfindungsgemäße Sensor ist definiert durch die Merkmale der Ansprüche 1-4. Nachfolgend wird ein Ausführungsbeispiel der Erfindung anhand einer Zeichnung beschrieben. In der Zeichnung zeigt:
Figur 1 den Zusammenhang zwischen der Zahl der Graphitteilchen und dem Sauerstoffgehalt (Sauerstoffaktivität aO).
Figur 2 den Zusammenhang zwischen der relativen Ausdehnung und dem Sauerstoffgehalt,
Figur 3 einen Querschnitt durch den erfindungsgemäßen Sensorkopf und
Figur 4 einen Teilschnitt durch eine weitere Ausführungsform des Sensors.

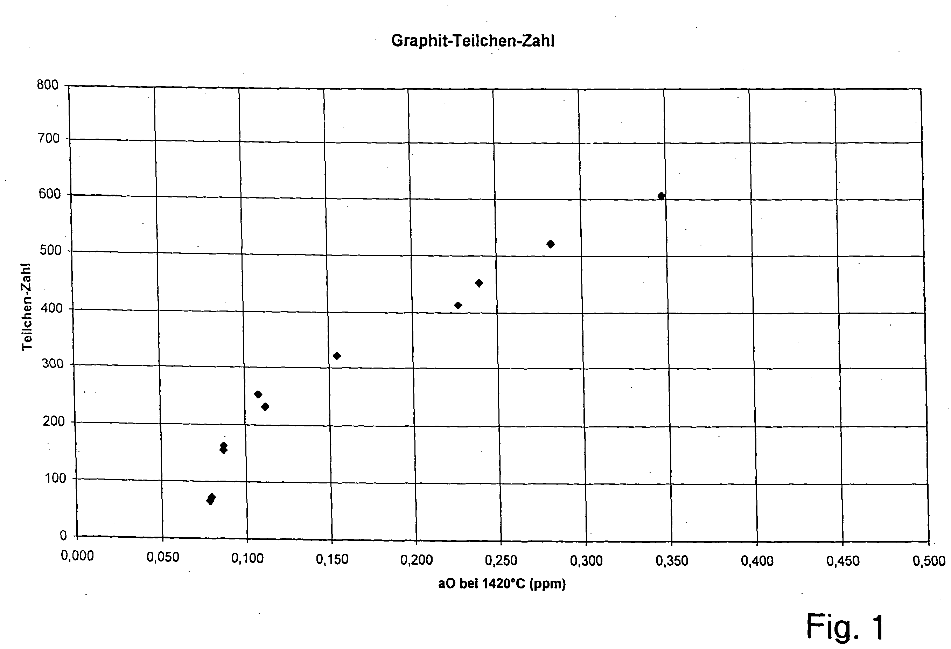

In Figur 1 ist zu sehen, dass die Zahl der Graphitteilchen mit zunehmendem Sauerstoffgehalt (Sauerstoffaktivität aO) steigt. Mit Hilfe der Regelung des Sauerstoffgehaltes über die Zugabe von Magnesium kann also die Zahl der Graphitteilchen eingestellt werden. Damit werden die Eigenschaften des Gusseisens bereits in der Schmelze gezielt beeinflusst. Eine maximale Nodularität tritt bei einer Sauerstoffaktivität zwischen etwa 0,10 und 0,12 ppm auf (gültig für 1.420°C). Fällt die Sauerstoffaktivität unter 0,10 ppm, reduziert sich die Nodularität.

Dies entspricht der bekannten Erfahrung aus der Gießereipraxis, dass ein zu hoher Magnesiumanteil negative Auswirkungen auf die Nodularität hat.

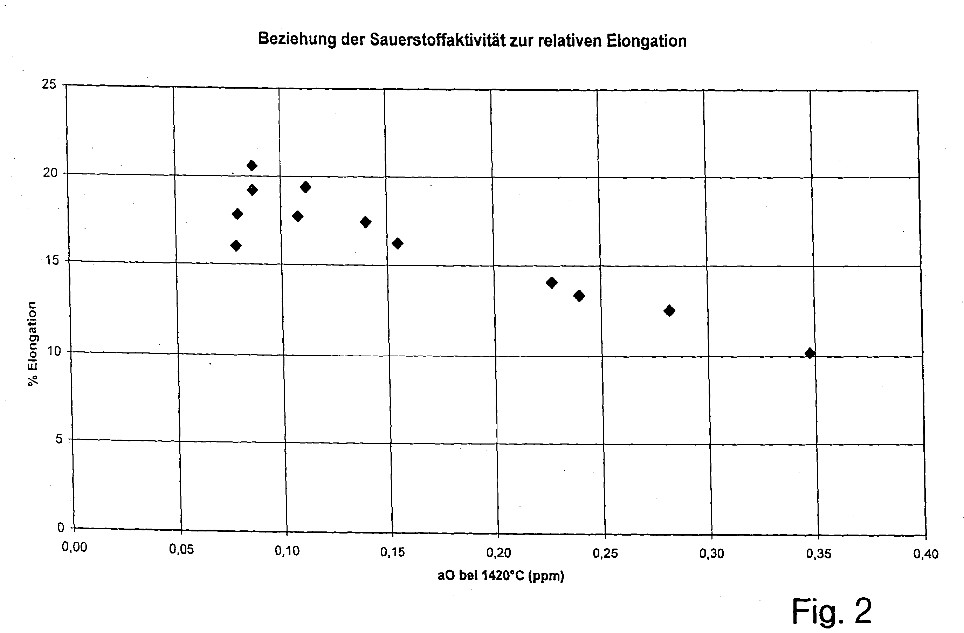

Figur 2 zeigt den Zusammenhang zwischen der relativen Ausdehnung des Gusseisens und dem Sauerstoffgehalt. Ein Maximum der Ausdehnung (Elongation) ist bei etwa 0,08 ppm erkennbar. Bei niedrigerer Sauerstoffaktivität ist die Dehnung geringfügig kleiner, vermutlich wegen der geringeren Nodularität. Übersteigt die Sauerstoffaktivität den optimalen Wert, kommt es zu einer stetigen Verringerung der Dehnung. Die Graphik zeigt, dass es möglich ist, durch die Einstellung des Sauerstoffgehaltes in der Gusseisenschmelze durch Zugabe von Magnesium die relative Ausdehnung des Gusseisens zu beeinflussen.

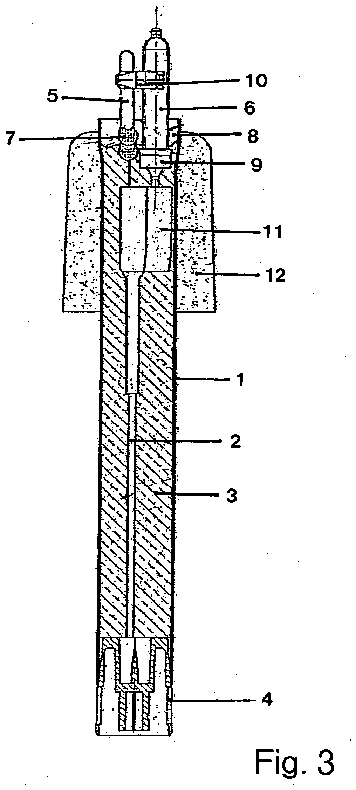

In Figur 3 ist ein erfindungsgemäßer Sensor dargestellt. In einem Metallrohr 1 sind die elektrischen Leitungen 2 (Cu/CuNi/Leiter) in einer Sandfüllung 3 angeordnet. Über das Verbindungsstück 4 werden die elektrischen Leitungen mit einer Lanze oder einem anderem Halter und des Weiteren mit einer Auswertereinheit verbunden. Das andere Ende der Leitungen 2 ist mit einem Thermoelement 5 und der elektrochemischen Messzelle 6 verbunden. Die elektrochemische Messzelle 6 weist ein Festelektrolytröhrchen (ZrO₂-Zelle) mit einem Stahlschockschild als äußere Hülle auf. Die ZrO₂-Zelle weist an ihrer äußeren Oberfläche eine Schicht aus Zirkondioxid auf, die mit 5 Gew.-% Kalziumoxid stabilisiert ist. Diese Schicht ist etwa 40 µm dick. In der Zeichnung ist sie nicht Im Einzelnen dargestellt, da Festelektrolytröhrchen grundsätzlich bekannt sind.

Das Thermoelement 5 ist in einem Thermoelement-Dichtungszement 7 fixiert. Die Messzelle 6 ist ebenfalls in einem Zement 8 fixiert, ihr im Inneren des Sensors angeordnetes Ende ist mit einem Dichtungsstöpsel 9 geschlossen, durch den die elektrischen Kontakte herausgeführt sind. Die beiden Sensorelemente 5; 6 sind mittels eines Plastikclips 10 miteinander verbunden. Durch das thermisch isolierende Teil 11 hindurch werden die Leitungen durch das Innere des Metallrohres 1 geführt. An dem Eintauchen des Sensors ist ein Sandkörper 12 an der Außenseite des Metallrohres 1 angeordnet, um dieses zu schützen.

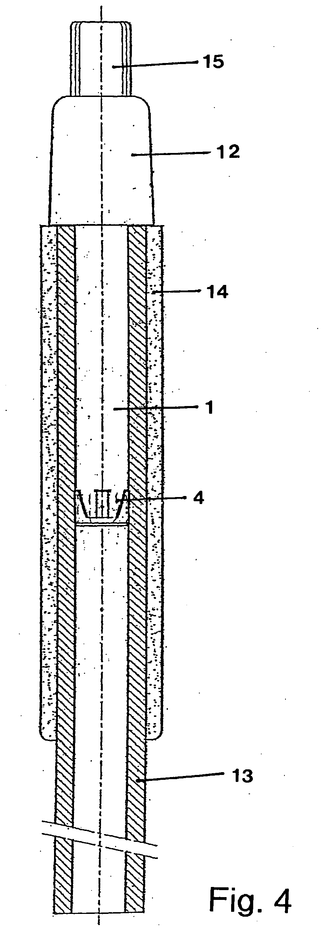

Figur 4 zeigt eine ähnliche Anordnung, bei der die Kontaktierung des Sensors im Trägerrohr 13 dargestellt ist. Das Trägerrohr 13 ist aus Pappe gebildet und an seiner vorderen, dem Sandkörper 12 zugewandten Seite von einem Spritzschutzrohr 14 umgeben, welches aus Gießereisand oder Zement gebildet ist.

Die Sensorelemente 5; 6 selbst sind zum Schutz beim Transport und beim Eintauchen in die Schmelze zunächst mit einer Metallkappe 15 umgeben, die beim bzw. nach dem Eintauchen des Sensors in die Metallschmelze schmilzt und die Sensorelemente 5; 6 freigibt.

## Patentansprüche

1. Sensor zum Messen des Sauerstoffgehaltes in Gusseisenschmelzen mit einer elektrochemischen Messzelle, die ein Festelektrolytröhrchen aufweist, **dadurch gekennzeichnet, dass** auf der nach außen gerichteten Oberfläche des Festelektrolytröhrchens eine Schicht aus Zirkondioxid aufgebracht ist, die mit 4 bis 6 Gew.-% Kalziumoxid stabilisiert ist und dass die Schicht plasmagespritzt ist.

2. Sensor nach Anspruch 1, **dadurch gekennzeichnet, dass** die Schicht 30 bis 50 µm, insbesondere 40 µm dick ist.

3. Sensor nach einem der Ansprüche 1 oder 2, **dadurch gekennzeichnet, dass** das Festelektrolytröhrchen als Zirkondioxidröhrchen ausgebildet ist.

4. Sensor nach Anspruch 3, **dadurch gekennzeichnet, dass** das Zirkondioxidröhrchen mit 2 Gew.-% Magnesiumoxid stabilisiert ist.

## Claims

1. Sensor for measuring the oxygen content in cast iron melts comprising an electrochemical measuring cell that comprises a solid electrolyte tube, **characterised in that** the outward facing surface of the solid electrolyte tube has a layer of zirconium dioxide applied to it that is stabilised by 4 to 6% by weight of calcium oxide, and **in that** the layer is plasma-sprayed.

2. Sensor according to claim 1, **characterised in that** the layer is 30 to 50 µm, in particular 40 µm, in thickness.

3. Sensor according to either one of the claims 1 or 2, **characterised in that** the solid electrolyte tube is embodied in the form of a zirconium dioxide tube.

4. Sensor according to claim 3, **characterised in that** the zirconium dioxide tube is stabilised by 2% by weight of magnesium oxide.

## Revendications

1. Sonde pour mesurer la teneur en oxygène dans des masses fondues de fonte avec une cellule de mesure électrochimique, qui comporte un tube en électrolyte solide, **caractérisée en ce que** la surface dirigée vers l'extérieur du tube en électrolyte solide comporte une couche de dioxyde de zirconium, qui est stabilisée avec 4 à 6 % poids d'oxyde de calcium et qui est traitée par une projection de plasma.

2. Sonde selon la revendication 1, **caractérisée en ce que** la couche présente une épaisseur de 30 à 50 µm, notamment 40 µm.

3. Sonde selon l'une des revendications 1 ou 2, **caractérisée en ce que** le tube en électrolyte solide est formé en tant que tube en dioxyde de zirconium.

4. Sonde selon la revendication 3, **caractérisée en ce que** le tube en dioxyde de zirconium est stabilisé avec 2 % poids de magnésie.
